# EUROPEAN PATENT SPECIFICATION

(11) **EP 4 247 796 B1**
(45) Date of publication and mention of the grant of the patent: **03.09.2025**
(21) Application number: 21824090.1
(22) Date of filing: 18.11.2021
(51) Int. Cl.: C07D 249/10, C07D 303/08, C07C 243/18

(54) **PROCESS FOR THE PREPARATION OF PROTHIOCONAZOLE AND INTERMEDIATES THEREOF**
VERFAHREN ZUR HERSTELLUNG VON PROTHIOCONAZOL UND ZWISCHENPRODUKTEN DAVON
PROCÉDÉ DE PRÉPARATION DE PROTHIOCONAZOLE ET SES INTERMÉDIAIRES

(30) Priority: 19.11.2020 US 202063115631 P
(43) Date of publication of application: 27.09.2023
(73) Proprietor: ADAMA MAKHTESHIM LTD., 8410001 Beer Sheva (IL)
(72) Inventor: RAGUAN PEN, Elizabetha, 8423658 Beer-Sheva (IL); MONHAIT, Avi, 5228104 Ramat-Gan (IL); EZION, Amir, 8496500 Omer (IL); GELMAN, Elijah, 8451598 Beer-Sheva (IL); NISENBLAT, Ron, 8460806 Beer-Sheva (IL)
(74) Representative: Modiano, Gabriella Diana
(86) International application number: PCT/IL2021/051375
(87) International publication number: WO 2022/107139

(56) References cited:
- WO-A1-2019/123368
- WO-A1-2020/134104
- WO-A1-2021/074739
- DE-A1- 4 030 039
- US-A1- 2001 011 138
- US-B2- 6 559 317

## Description

### FIELD OF THE INVENTION

The present invention relates to the field of organic synthesis, specifically to an improved method for the preparation of prothioconazole and its intermediates 2-(1-chloro-cycloprop-1-yl)-3-(2-chlorophenyl)-2-hydroxy-propyl-1-hydrazine and 2-[2-(1-chlorocyclopropyl)-3-(2- chlorophenyl)-2-hydroxypropyl]-1,2,4-triazolidine-3-thione.

### BACKGROUND PRIOR ART

Prothioconazole, 2-[(2RS)-2-(1-chlorocyclopropyl)-3-(2-chlorophenyl)-2-hydroxypropyl]-2H-1,2,4-triazole-3(4H)-thione, is a fungicide used to treat infected crops, first described in US 5,789,430. The structure is reproduced below:

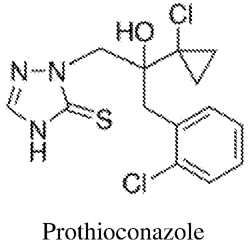

Key intermediates in the preparation of prothioconazole are 2-(1-chlorocycloprop-1-yl)-3-(2-chlorophenyl)-2-hydroxypropyl-1-hydrazine and 2-[2-(1-chlorocyclopropyl)-3-(2-chlorophenyl)-2-hydroxypropyl]-1,2,4-triazolidine-3-thione. Their preparation still presents many challenges as it can be deduced from the problems of the different strategies disclosed in the prior art.

DE4030039A1 teaches the preparation of 2-(1-chloro-cyclopropyl)-1-(2-chlorophenyl)-3-(1,2,4-triazol-1-yl)propan-2-ol starting from 2-(1-chlorocyclopropyl)-2-(2-chlorobenzyl) oxirane using a two stage process. For the first stage, the oxirane is reacted with hydrazine in the presence of a solvent and, optionally, a phase transfer catalyst to provide 2-(1-chlorocycloprop-1-yl)-3-(2-chlorophenyl)-2-hydroxypropyl-1-hydrazine. The solvents used by DE4030039A1 for this reaction are alcohols and ethers, such as methanol, ethanol, n-propanol, isopropanol, n-butanol, diethyl ether, dioxane and tetrahydrofuran. For the second stage, DE4030039A1 teaches the same solvents. The hydrazine derivative is reacted with formamidine acetate in the presence of a phase transfer catalyst to provide 2-(1-chlorocyclopropyl)-1-(2-chlorophenyl)-3-(1,2,4-triazol-1-yl)propan-2-ol. The subsequent transformation of this intermediate into prothioconazole is achieved by reactions that involve the use of sulfur and, optionally butyllithium.

A similar teaching is found in WO 2021/074739. According to the description, the solvent used to obtain the 2-(1-chlorocycloprop-1-yl)-3-(2-chlorophenyl)-2-hydroxypropyl-1-hydrazine can be C1-C4 alcohols selected from methanol, ethanol, isopropanol and t - butanol; cyclohexanol, toluene, acetonitrile (ACN), N, N-dimethyl formamide (DMF) and sulfolane. In the example shown on page 11 n-butanol is however used, which is partially soluble in water (about 73 g/L at 25°C).

In order to solve the above and other problems to reach 2-[2-(1-chlorocyclopropyl)-3-(2-chlorophenyl)-2-hydroxypropyl]-1,2,4-triazolidine-3-thione, US 2001/0011138 discloses its preparation directly from 2-(1-chlorocycloprop-1-yl)-3-(2-chlorophenyl)-2-hydroxypropyl-1-hydrazine by reacting the latter with a thiocyanate in toluene (paragraph [0089]). For the preparation of the hydrazine derivative used as starting material, US 2001/0011138 relies on the teachings of DE4030039A1.

An important problem of the two prior methods is the instability of 2-(1-chlorocycloprop-1-yl)-3-(2-chlorophenyl)-2-hydroxypropyl-1-hydrazine. US 6,559,317 seeks to solve this problem by generating its hydrochloric salt, a far more stable compound. US 6,559,317 teaches the preparation of the hydrochloric salt of the hydrazine derivative by first reacting 2-(1-chlorocyclopropyl)-2-(2-chlorobenzyl)oxirane with hydrazine in a mixture of toluene and acrylonitrile and then passing hydrochloric gas through the mixture. WO 2019/123368 further improves the conditions disclosed in US 6,559,317 and teaches against the use of toluene for the formation of 2-(1-chlorocycloprop-1-yl)-3-(2-chlorophenyl)-2-hydroxypropyl-1-hydrazine. According to WO 2019/123368, 1-chloro-2-(1-chlorocyclopropyl)-3-(2-chlorophenyl)propan-2-ol is a more advantageous starting material than 2-(1-chlorocyclopropyl)-2-(2-chlorobenzyl)oxirane.

### SUMMARY OF THE INVENTION

In the search for a more efficient process the inventors have designed a strategy that is surprisingly more efficient than those of the prior art, and provides 2-[2-(1-chlorocyclopropyl)-3-(2-chlorophenyl)-2-hydroxypropyl]-1,2,4-triazolidine-3-thione in good yield and purity.

The first aspect disclosed herein is therefore a process for the preparation of a mixture comprising the neutral form of a hydrazine derivative of Formula (I) and a first solvent or mixture of solvents, the process comprising (i) a two-phase reaction between an oxirane of Formula (II) and hydrazine in the presence of water and the first solvent or mixture of solvents, wherein said first solvent or mixture of solvents consist of one or more water-insoluble solvents;
wherein the compounds of Formula (I) and Formula (II) are the following:

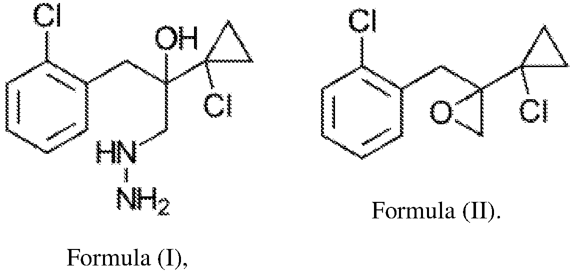

A second aspect is a process for the preparation of a thiono derivative of Formula (III) comprising
(i) reacting an oxirane of Formula (II) with hydrazine, in the presence of water and a first solvent or mixture of solvents, wherein said first solvent or mixture of solvents consist of one or more water-insoluble solvents, to provide a mixture comprising the neutral form of a hydrazine derivative of Formula (I) and the first solvent or mixture of solvents; and
(ii) reacting the mixture comprising the neutral form of a hydrazine derivative of Formula (I) and the first solvent or mixture of solvents resulting from step (i) with formaldehyde and a thiocyanate of formula X-SCN, wherein X is an alkaline cation or ammonium, , in the presence of an acid (e.g. phosphoric acid, HCl, sulfuric, formic acid, acetic acid), and in the presence of a second solvent or mixture of solvents and water to provide the thiono derivative of Formula (III), wherein said second solvent or mixture of solvents consist of one or more water-insoluble solvents;
and wherein the solution comprising the neutral form of the hydrazine derivative of Formula (I) and the first solvent or mixture of solvents resulting from step (i) is fed into step (ii) without isolation as a mixture comprising between 1 %w/w and 99 %w/w of the hydrazine derivative of Formula (I), with respect to the total weight of the solution; wherein the compounds of Formula (I), Formula (II) and Formula (III) are the following:

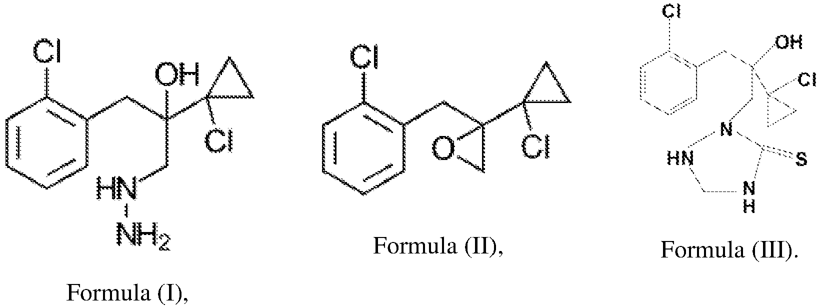

Therefore, by using only solvents that have low solubility in water (referred to as the "first solvent or mixture of solvents") in step (i) achieved a one-pot process starting from the oxirane derivative of Formula (II) all the way to the thiono derivative of Formula (III), that is 2-[2-(1-chlorocyclopropyl)-3-(2-chlorophenyl)-2-hydroxypropyl]-1,2,4-triazolidine-3-thione, without the need of isolating the intermediate hydrazine derivative of Formula (I), that is 2-(1-chloro-cycloprop-1-yl)-3-(2-chlorophenyl)-2-hydroxypropyl-1-hydrazine.

It is not only that the hydrazine derivative of Formula (I) can be used without isolation, there is not even the need to evaporate the solvent, and the mixture can be used as it is directly for the next step, or after simple conditioning steps (e.g. washing or filtrating). This notably simplifies the industrial process.

Other processes in the prior art use solvents that are partially soluble in water, such as methanol. These solvents drag some of the product into the water phase and therefore have to be evaporated, a procedure that in the presence of hydrazine causes many safety concerns that limit or even prevents industrial application. Such evaporation is also necessary to recycle the hydrazine.

Therefore, a further aspect is a mixture comprising a first solvent or mixture of solvents and between 1 %w/w and 99 %w/w of the hydrazine derivative of Formula (I), wherein the first solvent or mixture of solvents consist of one or more water-insoluble solvents. A further aspect is the use to prepare prothioconazole of a mixture comprising between 1 %w/w and 99 %w/w of a hydrazine derivative of Formula (I) in a first solvent or mixture of solvents, wherein said first solvent or mixture of solvents consist of one or more water-insoluble solvents.

The processes described herein provide the thiono derivative of Formula (III) in high yield and purity. This result is surprising given the instability of the hydrazine derivative of Formula (I). To solve this problem the prior art has suggested different solvent systems for the reactions or the preparation of the corresponding hydrochloric salt, even at the expense of introducing additional steps. The processes disclosed herein solve the stability problem by preparing a mixture wherein the hydrazine derivative of Formula (I) is sufficiently stable, while allowing to directly proceed to the next stage with the crude product. The use of water-insoluble solvents also avoids other solvents recommended in the prior art, such as methanol, which create safety issues during evaporation. Therefore, contrary to DE4030039A1, the processes disclosed herein use water-insoluble solvents (e.g. toluene) as a solvent in step (i), allowing the preparation of a crude solution of 2-(1-chlorocycloprop-1-yl)-3-(2-chlorophenyl)-2-hydroxypropyl-1-hydrazine (the hydrazine derivative of Formula (I)) typically in toluene that can be used directly in the next step.

Neither does US 2001/0011138 teach the use of water-insoluble solvents, such as toluene, for obtaining the hydrazine derivative of Formula (I). In fact, US 2001/0011138 teaches that the preferred starting material for obtaining this intermediate is not the oxirane derivative of Formula (II), but the hydroxy-chloride derivative 1-chloro-2-(1-chlorocyclopropyl)-3-(2-chlorophenyl)propan-2-ol. Example 2 teaches a one-pot process starting from this hydroxy-chloride, not from the oxirane derivative of Formula (II), to prepare the thiono derivative of Formula (III). The first step of the process uses no solvent, and the second step of the process takes place in methyl tert-butyl ether, not toluene, providing an overall poor yield (47% from the hydroxy-chloride). The yields of the process disclosed herein are higher (almost 75%). Therefore, US 2001/0011138 fails to teach the use of toluene in the production of the hydrazine derivative of Formula (I) or the possibility of improving the yields using a one-pot process starting from the oxirane derivative of Formula (II) and using a water-insoluble solvent all the way to the thiono derivative of Formula (III).

The process disclosed herein by the inventors also avoids the use of the hydrochloric salt of the hydrazine derivative of Formula (I), as taught in US 6,559,317 or WO 2019/123368, notably simplifying the process. Although preparing the more stable salt may seem attractive, it requires two additional steps: the formation of the salt itself, which uses gaseous hydrochloric acid, and the subsequent neutralization with a base.

Thus, a further aspect is a process for the preparation of prothioconazole comprising
(i) reacting an oxirane of Formula (II) with hydrazine, in the presence of water and a first solvent or mixture of solvents, wherein said first solvent or mixture of solvents consist of one or more water-insoluble solvents, to provide a mixture comprising the neutral form of a hydrazine derivative of Formula (I) and the first solvent or mixture of solvents;
(ii) reacting the first solvent or mixture of solvents and the hydrazine derivative of Formula (I) resulting from step (i) with formaldehyde and a thiocyanate of formula X-SCN, wherein X is an alkaline cation or ammonium, in the presence of an acid (e.g. phosphoric acid, HCl, sulfuric, formic acid, acetic acid), and in the presence of a second solvent or mixture of solvents and water to provide a thiono derivative of Formula (III), wherein said second solvent or mixture of solvents comprises one or more water-insoluble solvents; wherein the mixture comprising the neutral form of the hydrazine derivative of Formula (I) and the first solvent or mixture of solvents resulting from step (i) is fed into step (ii) without purification as a mixture comprising between 1 %w/w and 99 %w/w of the hydrazine derivative of Formula (I), with respect to the total weight of the mixture; and
(iii) transforming the thiono derivative of Formula (III) into prothioconazole; wherein the compounds of Formula (I), Formula (II) and Formula (III) are the following:

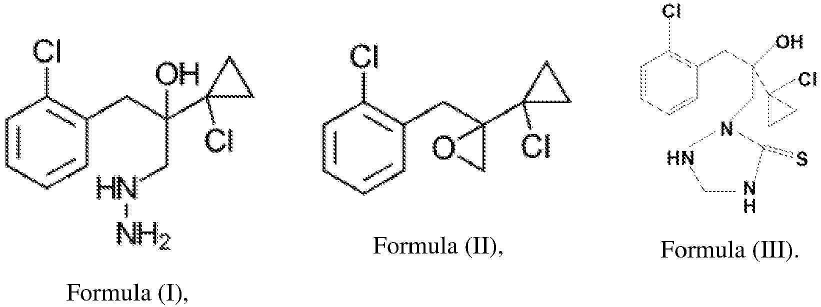

### DETAILED DESCRIPTION OF THE INVENTION

### Preparation of hydrazine derivatives of Formula (I)

The starting material used in the processes disclosed herein is the oxirane derivative of Formula (II), which can be obtained by methods already taught in the art, for example in DE4030039A1 or in US 5,097,047. See for example columns 10 and 11 of US 5,097,047. For example, the oxirane derivative of Formula (II) can be obtained by intracyclization of the corresponding hydroxy-chloride in the presence of an acid binder, as disclosed in paragraphs [0078]-[0081] of US 2001/0011138. Alternatively, the intramolecular cyclization can be performed using a base as taught in example 1.b) of DE4030039A1.

The one or more water-insoluble solvents used in the process of the invention allow the two-phase reaction to proceed efficiently and in good yield. Further, the separation of the water phase and the organic phase once the reaction has finished is simple and effective. The aqueous phase retains unreacted hydrazine and other impurities, and the organic phase is a mixture comprising the hydrazine derivative of Formula (I) in neutral form and the first solvent or mixture of solvents that are water-insoluble. This organic phase is sufficiently pure to directly proceed to the next step of the process, and does not require any evaporation steps in the presence of hydrazine. Some conditioning processes, such as filtration or washing (e.g. with a neutral of basic aqueous solution) are optional. Any solvent that is insoluble in water is suitable for the process of the invention. It is preferred that the one or more water-insoluble solvents comprise one or more solvents selected from the group consisting of a C₇-C₂₀ alcohol, a C₆-C₂₀ aromatic hydrocarbon and mixtures thereof, preferably a C₇-C₁₂-aromatic hydrocarbon selected from the group consisting of toluene, xylene and mixtures thereof.

Thus, the process of the invention preferably comprises (i) a two-phase reaction between an oxirane of Formula (II) and hydrazine, in the presence of water and the first solvent or mixture of solvents, wherein said first solvent or mixture of solvents consist of one or more water-insoluble solvents selected from the group consisting of a C₇-C₂₀ alcohol, a C₆-C₂₀ aromatic hydrocarbon and mixtures thereof.

Typically, the process comprises (i) a two-phase reaction between an oxirane of Formula (II) and hydrazine, in the presence of water and the first solvent or mixture of solvents, wherein said first solvent or mixture of solvents consist of one or more water-insoluble solvents comprising a C₆-C₂₀ or C₇-C₂₀ aromatic hydrocarbon.

The water-insoluble solvent typically comprises toluene and can be the only water-insoluble solvent used.

The process preferably takes place in the presence of one, that is, a single, water-insoluble solvent. Therefore, the process of the invention typically comprises (i) a two-phase reaction between an oxirane of Formula (II) and hydrazine, in the presence of water and the first solvent, wherein said first solvent consists of one water-insoluble solvent, preferably wherein the water-insoluble solvent consists of toluene.

Water is not necessarily added as such, but typically comes with the reagents, mainly with the hydrazine.

The preparation of the hydrazine derivative of Formula (I) may therefore proceed by contacting the oxirane derivative of Formula (II) with hydrazine using toluene as the only water-insoluble solvent.

Hydrazine can be used in any suitable form, typically hydrazine hydrate. Hydrazine is typically fed into the reaction mixture as an aqueous solution, for example, a 20 to 65%w/w aqueous solution, for example, it can be added as hydrazine hydrate 100 % (i.e. 65 %w/w hydrazine). It can be added alone or together with a phase transfer catalyst. The hydrazine is typically used in molar excess with respect to the oxirane derivative of Formula (II). The inventors have found that the process disclosed herein requires a lower excess of hydrazine, especially when using a phase transfer catalyst, which is another advantage of the process. Thus, the proportion between the hydrazine and the oxirane of Formula (II) is typically comprised between 1:1 and 20:1, for example, between 3:1 and 20:1, or between 15:1 and 10:1, preferably, between 8:1 to 7:1. Said excess of hydrazine can be recycled into the reaction. If no recycling is desired, the process can function even at lower excesses of hydrazine, for example, the proportion between the hydrazine and the oxirane of Formula (II) can be comprised between 1:1 and 5:1, or between 1.05:1 and 3:1.

The process may use a phase transfer catalyst, such as ammonium or phosphonium compounds. Phase transfer catalysts can be selected from the group consisting of tetraalkyl ammonium salts, trialkyl aralkylammonium salts or tetraalkyl-phosphonium salts such as tetrabutylammonium bromide or chloride, methyl trioctylammonium bromide, methyl tributylammonium chloride, triethyl benzyl ammonium bromide or chloride, and benzyl dodecyl dimethyl ammonium chloride (known as Zephirol^{®}). Numerous brands exist such as the Aliquat^{®} series. The phase-transfer catalysts are generally used in aqueous or alcoholic solutions. The phase transfer catalyst can be a single species such as methyl tributylammonium chloride (Aliquat^{®} 175) or a mixture such as Aliquat^{®} 336 (Stark's catalyst), which is quaternary ammonium salt that contains a mixture of octyl and decyl chains.

The reaction can proceed in the presence of a wide range of phase transfer catalyst proportions. Preferably, the molar percentage of phase transfer catalyst with respect to the oxirane is between 0.1 and 50 mol%, preferably between 0.5 and 40 mol%. It has been found that the use of a solid PTC is surprisingly efficient and can reduce by more than half the total amount of PTC used. Thus, when using solid PTC its amount used with respect to the oxirane is between 0.1 and 50 mol%, preferably between 1 and 40 mol%, or even between 1 and 20 mol%.

The reaction typically proceeds at atmospheric pressure, but other pressures can be used. Temperature is typically required to proceed in a reasonable time and the reaction is usually maintained at reflux, typically at a temperature comprised between 20°C and 150°C, for example between 40°C and 150°C, for example between 20°C and 100°C, for example between 50°C and 95°C.

Therefore, the process is typically a two-phase system. Once the reaction is complete, the process can comprise an additional step (ia) wherein the two phases of the two-phase reaction mixture are separated to create a first current and a second current, wherein the first current comprises aqueous unreacted hydrazine (and, optionally, also the phase transfer catalyst), and the second current comprises the first solvent or mixture of solvents (e.g toluene) and the hydrazine derivative of Formula (I). This separation can be done at a temperature comprised between 20°C and 80°C, preferably between 40°C and 70°C. The first current comprising unreacted hydrazine (and, optionally, also the phase transfer catalyst) can be directly recycled for subsequent reactions, especially when a large excess is used, optionally after being mixed with fresh hydrazine. For example, the process can use a proportion between the hydrazine and the oxirane of Formula (II) comprised between 3:1 and 20:1 or between 4:1 and 20:1, wherein the excess of hydrazine is recycled for subsequent reactions.

The reaction can take place at a gentle reflux. The material distilled can be recovered in a separate current or condensed back into the reaction. Thus, the evaporation of water and hydrazine from the reaction mixture can start before the reaction is complete without interfering in the final yield or conversion.

The second current comprising toluene and the hydrazine derivative of Formula (I) can be optionally washed, for example, at least one time with a neutral or basic solution, and then be used directly in the next step to obtain the thiono derivative of Formula (III). This hydrazine derivative of Formula (I) is sufficiently stable in the first solvent or mixture of solvents to be used in subsequent steps without any change of solvent (no evaporation is necessary), which is a very efficient strategy. This solution can be obtained at different concentrations, typically between 1 %w/w and 99 %w/w of the hydrazine derivative of Formula (I) and toluene, for example between 5 %w/w and 95 %w/w, preferably between 10 %w/w and 80 %w/w, preferably between 12 %w/w and 60 %w/w, more preferably between 15 %w/w and 50 %w/w, preferably between 15 %w/w and 40 %w/w, even more preferably between 20 %w/w and 30 %w/w of the hydrazine derivative of Formula (I) in toluene.

### One-not preparation of a compound of Formula (III)

As mentioned above, a further aspect is a comprising
(i) reacting an oxirane of Formula (II) with hydrazine, in the presence of water and a first solvent or mixture of solvents, wherein said first solvent or mixture of solvents consist of one or more water-insoluble solvents, to provide a mixture comprising the neutral form of a hydrazine derivative of Formula (I) and the first solvent or mixture of solvents; and
(ii) reacting the mixture comprising the neutral form of a hydrazine derivative of Formula (I) and the first solvent or mixture of solvents resulting from step (i) with formaldehyde and a thiocyanate of formula X-SCN, wherein X is an alkaline cation or ammonium, in the presence of an acid (e.g. phosphoric acid, HCl, sulfuric, formic acid, acetic acid), and in the presence of a second solvent or mixture of solvents and water to provide the thiono derivative of Formula (III), wherein said second solvent or mixture of solvents consist of one or more water-insoluble solvents.

The solution comprising the neutral form of the hydrazine derivative of Formula (I) and the first solvent or mixture of solvents resulting from step (i) is fed into step (ii) without isolation as a mixture comprising between 1 %w/w and 99 %w/w of the hydrazine derivative of Formula (I), with respect to the total weight of the solution.

Optionally, the mixture of hydrazine derivative of Formula (I) in the water-insoluble solvent resulting from step (i) is washed with an aqueous solution before use. Thus, the process for the preparation of a thiono derivative of Formula (III) may comprise
(i) reacting an oxirane of Formula (II) with hydrazine, in the presence of water and a first solvent or mixture of solvents, wherein said first solvent or mixture of solvents consist of one or more water-insoluble solvents, to provide a mixture comprising the neutral form of a hydrazine derivative of Formula (I) and the first solvent or mixture of solvents;
(ia) separating the two phases of the two-phase reaction mixture to create a first current and a second current, wherein the first current comprises aqueous unreacted hydrazine, and the second current comprises the first solvent or mixture of solvents and the hydrazine derivative of Formula (I); and
(ii) reacting the second current with formaldehyde and a thiocyanate of formula X-SCN, wherein X is an alkaline cation or ammonium, in the presence of an acid (e.g. phosphoric acid, HCl, sulfuric, formic acid, acetic acid), and in the presence of a second solvent or mixture of solvents and water to provide a thiono derivative of Formula (III), wherein said second solvent or mixture of solvents consist of one or more water-insoluble solvents;
wherein the second current is used for step (ii) without isolation and comprises a mixture of between 1 %w/w and 99 %w/w of the hydrazine derivative of Formula (I) in the first solvent or mixture of solvents, with respect to the total weight of the second current.

Step (i) of this process proceeds as described above in the previous section. Step (ii) may also use a second solvent or mixture of solvents that is water-insoluble, and requires formaldehyde and a thiocyanate for completion, and optionally an acid, typically an inorganic acid, such as phosphoric acid, HCl, sulfuric, or organic acids, such as formic acid or acetic acid.

It is preferred that the solvent used in step (ii) is the same as in step (i), that is, it is preferred that the first solvent or mixture of solvents is the same as the second solvent or mixture of solvents. Step (ii) typically proceeds at atmospheric pressure, but other pressures can be used. Temperature is typically required to proceed in a reasonable time and the reaction is usually maintained at a temperature comprised between 20°C and 100°C, for example between 30°C and 80°C.

Again, there is no need to add water as such, which usually comes together with the reagents, formaldehyde, the thiocyanate and the acid.

The formaldehyde can be employed as paraformaldehyde, as gaseous formaldehyde or as Formalin solution (aqueous formaldehyde solution). Preference is given to using Formalin solution. The preferred thiocyanate is sodium thiocyanate. Both, formaldehyde and the thiocyanate are typically used in excess, for example from 1 to 2 equivalents of formaldehyde and/or from 1 to 2 equivalents of thiocyanate for every mol of hydrazine derivative of Formula (I). Typically, up to 1.5 equivalents of formaldehyde are used, preferably, between 1 and 1.4 or between 1 and 1.2.

The thiono derivative of Formula (III) obtained can be purified through the usual processes, such as chromatographic purification or recrystallization, for example, in the same water-insoluble solvent, e.g. toluene. The order of addition of formaldehyde, thiocyanate and acid is irrelevant.

In order to provide prothioconazole, the thiono derivative of Formula (III) must be oxidxidized or aromatized. This can be done by following processes already disclosed in the prior art, such as for example, WO 2019/123368 A1 (see pages 9 through 13). Exemplary methods to perform the oxidation involve the aromatization in the presence of an oxidizing agent in a suitable solvent or mixture of solvents. Alternatively, aromatization can be acid catalyzed in the presence of an oxidizing agent in a suitable solvent or mixture of solvents. Acids can be selected from the group consisting of hydrochloric acid, hydrobromic acid, acetic acid, benzoic acid, substituted benzoic acid, trifluoroacetic acid, and formic acid. Acids can also be Lewis acids such as, aluminium chloride, stannous chloride, stannic chloride, titanium tetrachloride, boron trifluoride diethyl etherate, boron THF complex, or zinc chloride. Oxidizing agents can be selected from the group consisting of hydrogen peroxide, m-chloro perbenzoic acid (ra-CPBA), oxone, tert- butyl hydrogen peroxide (TBHP), copper sulphate, sodium nitrite, t-butyl nitrite, Ferric Chloride (III) and tert- butyl nitrite.

### EXAMPLES

### Example 1: preparation of 2-(1-Chlorocyclopropyl)-1-(2-chlorophenyl)-3-hydrazino-2-propanol (hydrazine derivative of Formula (I))

An 8 liter reactor was vented with nitrogen, and then fed with toluene (2700 gr), 2-(1-chloro-cycloprop-1-yl)- 2-(2'-chloro-benzyl)-oxirane (1200 gr in 77.7 %w/w purity), Aliquat^{®} 175 (10 gr of a 75 %w/w methyltributylammonium chloride in water). The reactor was heated to 60°C and 1205 gr of hydrazine hydrate and additional 300 gr of Aliquat^{®} 175 (75 %w/w) were added. The temperature was raised to 90°C and the reaction mixture stirred for 5 hours. When more than 98.5% of the oxirane was consumed, the reaction was consider finished. The mixture was cooled to 60°C and the organic and aqueous phases separated. The aqueous phase containing unreacted hydrazine and Aliquat^{®} 175 was recycled for the next batch (this reduced in subsequent batches the addition of fresh hydrazine to 291 gr and of fresh Aliquat^{®} to 10 gr). The organic phase was washed with 2500 gr of an aqueous solution of sodium carbonate (10 %w/w). The organic phase was separated to provide a 24 %w/w toluene solution of the hydrazine derivative of Formula (I) (2-(1-chloro-cycloprop-1-yl)-3- (2-chloro-phenyl)-2-hydroxypropyl- 1-hydrazine), which was used directly in the next step (90% yield). This notably increases the yield disclosed in example 2 of US 2001/0011138 (47%), which in the case of the present invention is 70% from the oxirane derivative of formula (II).

### Example 2: preparation of 2-(1-Chlorocyclopropyl)-1-(2-chlorophenyl)-3-hydrazino-2-propanol (hydrazine derivative of Formula (I))

An 10 liter reactor was vented with nitrogen, and then fed with toluene (3120 gr), 2-(1-chloro-cycloprop-1-yl)- 2-(2'-chloro-benzyl)-oxirane (1310 gr), solid Aliquat^{®} 175 (207.76 gr of a 98 %w/w methyltributylammonium chloride). The reactor was heated to 95°C and 411.6 gr of hydrazine hydrate were added. the reaction mixture stirred for 5 hours. When more than 98.5% of the oxirane was consumed, the reaction was consider finished. The mixture was cooled to 60°C and washed with 1200 gr water twice and phases separated. The 24 %w/w toluene solution of the hydrazine derivative of Formula (I) (2-(1-chloro-cycloprop-1-yl)-3- (2-chloro-phenyl)-2-hydroxy-propyl- 1-hydrazine), was used directly in the next step (88% yield).

The use of the PTC in solid form allowed the reduction of the proportion needed with respect to the oxirane starting material. In Example 1 it was about 26 mol%, while in this example it was about 16 mol%. Also, the amount of hydrazine hydrate could be significantly reduced.

### Example 3: preparation of 2-(1-Chlorocyclopropyl)-1-(2-chlorophenyl)-3-hydrazino-2-propanol (hydrazine derivative of Formula (I))

A 250 liter reactor was vented with nitrogen, and then fed with toluene (70Kg) and 2-(1-chloro-cycloprop-1-yl)-2-(2'-chloro-benzyl)-oxirane (25 Kg in 77.7 %w/w purity). The reactor was heated to 80°C and 60 Kg of hydrazine hydrate were added. The temperature was raised to 95°C and after 4 hours water evaporated for 1.5 hours. When more than 98.5% of the oxirane was consumed, the reaction was consider finished. The mixture was cooled to 60°C and the organic and the aqueous phases separated. The aqueous phase containing unreacted hydrazine was recycled for the next batch. The organic phase was washed twice with 40Kg of water. The organic phase was separated to provide a 20 %w/w toluene solution of the hydrazine derivative of Formula (I) (2-(1-chloro-cycloprop-1-yl)-3-(2-chloro-phenyl)-2-hydroxy-propyl-1-hydrazine), which was used directly in the next step (90% yield).

### Example 4: preparation of 2-[2-(1-chlorocyclopropyl)-3-(2-chlorophenyl)-2-hydroxypropyl]-1,2,4-triazolidine-3-thione

A 250 liter reactor was nitrogen vented and charged at room temperature with 100Kg of the toluene solution from the previous step containing the (2-(1-chloro-cycloprop-1-yl)-3- (2-chloro-phenyl)-2-hydroxy-propyl- 1-hydrazine). The reactor was heated to 50°C and fed with 16 Kg of a 50 %w/w water solution of sodium thiocyanate over a period of 10 minutes and stirred for additional 15 minutes at 50°C. 6 Kg of formalin and 8 Kg of H₃PO₄ were added over a period of 4 hours at 50°C with vigorous stirring. The temperature was raised to 75°C and stirred for 1 hour to yield 132 Kg of crude solution of the thiono derivative of Formula (III). The reaction mixture was cooled, filtrated and dried. 21 Kg of the thiono derivative of Formula (III) were obtained in 98% purity.

### Example 5: preparation of 2-[2-(1-chlorocyclopropyl)-3-(2-chlorophenyl)-2-hydroxypropyl]-1,2,4-triazolidine-3-thione

The reactor was nitrogen vented and charged at room temperature with 3950 Kg of the toluene solution from the previous step comprising (2-(1-chloro-cycloprop-1-yl)-3- (2-chloro-phenyl)-2-hydroxy-propyl- 1-hydrazine). The reactor was heated to 50°C and fed with 758.4 Kg of a 50 %w/w water solution of sodium thiocyanate over a period of 10 minutes and stirred for additional 15 minutes at 50°C. 324Kg of formalin and 398Kg of H₃PO₄ were added over a period of 4 hours at 50°C with vigorous stirring. The temperature was raised to 60°C and stirred for 1 hour to yield 5430 Kg of crude solution of the thiono derivative of Formula (III). The reaction mixture was cooled and filtrated, and the thiono derivative of Formula (III) was recrystallized from toluene.

## Claims

1. A process for the preparation of a mixture comprising a) the neutral form of a hydrazine derivative of Formula (I) and b) a first solvent or mixture of solvents, the process comprising (i) a two-phase reaction between an oxirane of Formula (II) and hydrazine in the presence of water and the first solvent or mixture of solvents, wherein said first solvent or mixture of solvents consist of one or more water-insoluble solvents; and
wherein the compounds of Formula (I) and Formula (II) are the following:

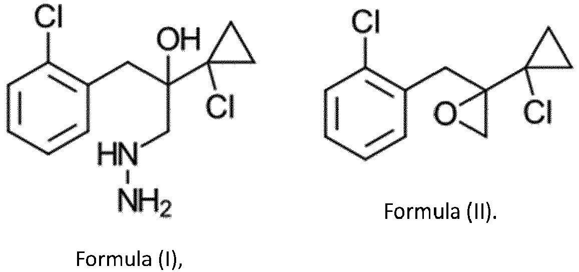

2. The process according to claim 1, wherein the one or more water-insoluble solvents comprise a C₇-C₁₂-aromatic hydrocarbon.

3. The process according to claim 2, wherein the C₇-C₁₂-aromatic hydrocarbon is selected from the group consisting of toluene, xylene and mixtures thereof.

4. The process according to any of the previous claims, wherein the reaction takes place in the presence of a phase transfer catalyst.

5. The process according to claim 4, wherein the phase transfer catalyst is selected from the group consisting of tetraalkyl ammonium salts, trialkyl aralkylammonium salts and tetraalkyl-phosphonium salts.

6. The process according to any of claims 4 or 5, wherein PTC is solid.

7. The process according to any of the previous claims, comprising an additional step (ia) wherein the two phases of the two-phase reaction mixture are separated to create a first current and a second current, wherein the first current comprises aqueous unreacted hydrazine, and the second current comprises said first solvent or mixture of solvents and the hydrazine derivative of Formula (I).

8. The process according to claim 7, wherein the step (ia) starts before the end of step (i).

9. The process according to any of claims 7 or 8, wherein the first current also comprises a phase transfer catalyst.

10. The process according to any of claims 7 to 9, wherein the second current comprising said first solvent or mixture of solvents and the hydrazine derivative of Formula (I) is fed into a reaction wherein the hydrazine derivative of Formula (I) is transformed into a thiono derivative of Formula (III)

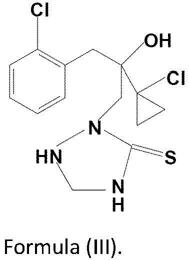

11. The process according to claim 10, wherein the hydrazine derivative of Formula (I) is treated in the presence of a second solvent or mixture of solvents with a) a thiocyanate of formula X-SCN, wherein X is an alkaline cation or ammonium, and b) formaldehyde, c) in the presence of an acid, wherein said second solvent or mixture of solvents comprises the first solvent or mixture of solvents.

12. Use to prepare prothioconazole of a mixture comprising between 1 %w/w and 99 %w/w of a hydrazine derivative of Formula (I) in a first solvent or mixture of solvents, wherein said first solvent or mixture of solvents consist of one or more water-insoluble solvents

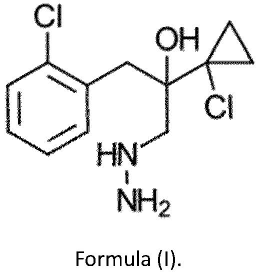

13. A process for the preparation of a thiono derivative of Formula (III) comprising
(i) reacting an oxirane of Formula (II) with hydrazine, in the presence of water and a first solvent or mixture of solvents, wherein said first solvent or mixture of solvents consist of one or more water-insoluble solvents, to provide a mixture comprising a) the neutral form of a hydrazine derivative of Formula (I) and b) the first solvent or mixture of solvents; and
(ii) reacting the mixture comprising a) the neutral form of a hydrazine derivative of Formula (I) and b) the first solvent or mixture of solvents resulting from step (i) with formaldehyde and a thiocyanate of formula X-SCN, wherein X is an alkaline cation or ammonium, in the presence of an acid, and in the presence of a second solvent or mixture of solvents and water, to provide the thiono derivative of Formula (III), wherein said second solvent or mixture of solvents consist of one or more water-insoluble solvents;
and wherein the solution comprising a) the neutral form of the hydrazine derivative of Formula (I) and b) the first solvent or mixture of solvents resulting from step (i) is fed into step (ii) without isolation as a mixture comprising between 1 %w/w and 99 %w/w of the hydrazine derivative of Formula (I), with respect to the total weight of the solution;
wherein the compounds of Formula (I), Formula (II) and Formula (III) are the following:

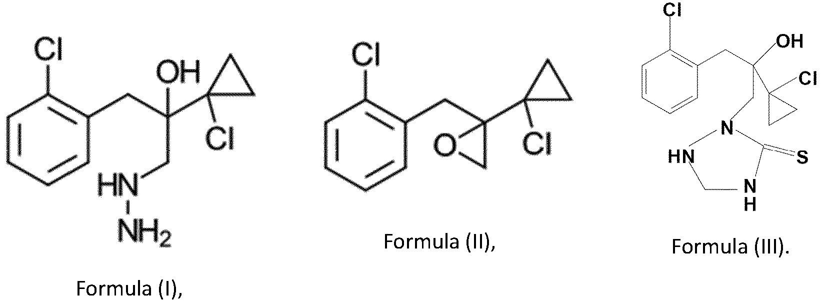

14. A process for the preparation of a thiono derivative of Formula (III), comprising
(i) reacting an oxirane of Formula (II) with hydrazine, in the presence of water and a first solvent or mixture of solvents, wherein said first solvent or mixture of solvents consist of one or more water-insoluble solvents, to provide a mixture comprising the neutral form of a hydrazine derivative of Formula (I) and the first solvent or mixture of solvents;
(ia) separating the two phases of the two-phase reaction mixture to create a first current and a second current, wherein the first current comprises aqueous unreacted hydrazine, and the second current comprises the first solvent or mixture of solvents and the hydrazine derivative of Formula (I); and
(ii) reacting the second current with formaldehyde and a thiocyanate of formula X-SCN, wherein X is an alkaline cation or ammonium, in the presence of an acid, and in the presence of a second solvent or mixture of solvents and water to provide a thiono derivative of Formula (III), wherein said second solvent or mixture of solvents consist of one or more water-insoluble solvents;
wherein the second current is used for step (ii) without isolation and comprises a mixture of between 1 %w/w and 99 %w/w of the hydrazine derivative of Formula (I) in the first solvent or mixture of solvents, with respect to the total weight of the second current;
wherein the compounds of Formula (I), Formula (II) and Formula (III) are the following:

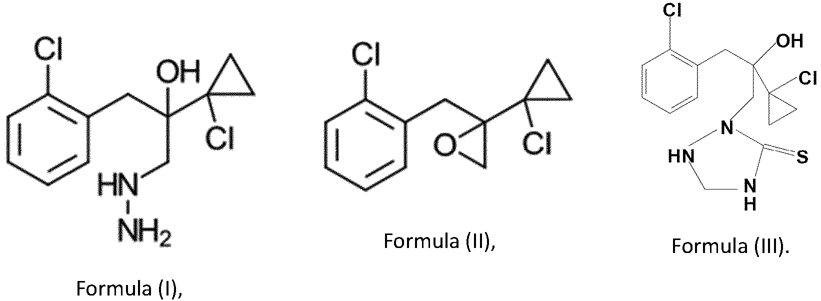

15. A process for the preparation of prothioconazole comprising
(i) reacting an oxirane of Formula (II) with hydrazine, in the presence of water and a first solvent or mixture of solvents, wherein said first solvent or mixture of solvents consist of one or more water-insoluble solvents, to provide a mixture comprising the neutral form of a hydrazine derivative of Formula (I) and the first solvent or mixture of solvents;
(ii) reacting the first solvent or mixture of solvents and the hydrazine derivative of Formula (I) resulting from step (i) with formaldehyde and a thiocyanate of formula X-SCN, wherein X is an alkaline cation or ammonium, in the presence of an acid, and in the presence of a second solvent or mixture of solvents and water to provide a thiono derivative of Formula (III), wherein said second solvent or mixture of solvents comprises one or more water-insoluble solvents;
wherein the mixture comprising the neutral form of the hydrazine derivative of Formula (I) and the first solvent or mixture of solvents resulting from step (i) is fed into step (ii) without purification as a mixture comprising between 1 %w/w and 99 %w/w of the hydrazine derivative of Formula (I), with respect to the total weight of the mixture; and
(iii) transforming the thiono derivative of Formula (III) into prothioconazole;
wherein the compounds of Formula (I), Formula (II) and Formula (III) are the following:

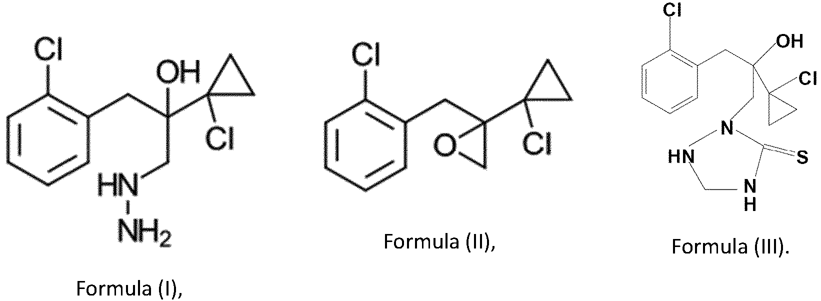

## Patentansprüche

1. Ein Verfahren zur Herstellung einer Mischung, die Folgendes umfasst: a) die neutrale Form eines Hydrazinderivats mit der Formel (I) und b) ein erstes Lösungsmittel oder eine Mischung von Lösungsmitteln, wobei das Verfahren Folgendes umfasst: (i) eine Zweiphasenreaktion zwischen einem Oxiran mit der Formel (II) und Hydrazin in Anwesenheit von Wasser und dem ersten Lösungsmittel oder der Mischung von Lösungsmitteln, wobei das erste Lösungsmittel oder die Mischung von Lösungsmitteln aus einem oder mehreren wasserunlöslichen Lösungsmitteln besteht und
wobei die Verbindungen mit der Formel (I) und der Formel (II) Folgende sind:

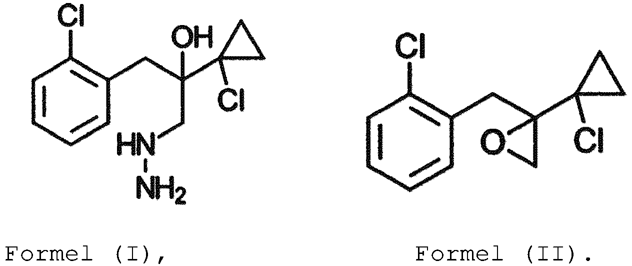

2. Das Verfahren gemäß Anspruch 1, worin die einen oder mehreren wasserunlöslichen Lösungsmittel einen aromatischen C₇-C₁₂-Kohlenwasserstoff umfassen.

3. Das Verfahren gemäß Anspruch 2, worin der aromatische C₇-C₁₂-Kohlenwasserstoff gewählt ist aus der Gruppe bestehend aus Toluen, Xylen und Mischungen davon.

4. Das Verfahren gemäß einem beliebigen der obigen Ansprüche, worin die Reaktion in Anwesenheit eines Phasentransferkatalysators stattfindet.

5. Das Verfahren gemäß Anspruch 4, worin der Phasentransferkatalysator gewählt ist aus der Gruppe bestehend aus Tetraalkylammoniumsalzen, Trialkylaralkylammoniumsalzen und Tetraalkylphosphoniumsalzen.

6. Das Verfahren gemäß einem beliebigen der Ansprüche 4 oder 5, worin der Phasentransferkatalysator fest ist.

7. Das Verfahren gemäß einem beliebigen der obigen Ansprüche, das einen zusätzlichen Schritt (ia) umfasst, worin die beiden Phasen der Zweiphasenreaktionsmischung getrennt werden, um einen ersten Strom und einen zweiten Strom zu erzeugen, wobei der erste Strom wässeriges, nicht umgesetztes Hydrazin umfasst und der zweite Strom das erste Lösungsmittel oder die Mischung von Lösungsmitteln und das Hydrazinderivat mit der Formel (I) umfasst.

8. Das Verfahren gemäß Anspruch 7, worin der Schritt (ia) vor dem Ende des Schritts (i) beginnt.

9. Das Verfahren gemäß einem beliebigen der Ansprüche 7 oder 8, worin der erste Strom auch einen Phasentransferkatalysator umfasst.

10. Das Verfahren gemäß einem beliebigen der Ansprüche 7 bis 9, worin der zweite Strom das erste Lösungsmittel oder die Mischung von Lösungsmitteln umfasst und das Hydrazinderivat mit der Formel (I) einer Reaktion zugeführt wird, worin das Hydrazinderivat mit der Formel (I) in ein Thionoderivat mit der Formel (III) umgewandelt wird

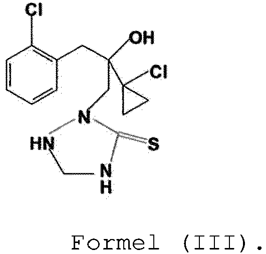

11. Das Verfahren gemäß Anspruch 10, worin das Hydrazinderivat mit der Formel (I) in Anwesenheit eines zweiten Lösungsmittels oder einer Mischung von Lösungsmitteln behandelt wird mit a) einem Thiocyanat mit der Formel X-SCN, worin X ein alkalisches Kation oder Ammonium ist, und b) Formaldehyd, c) in Anwesenheit einer Säure, wobei das zweite Lösungsmittel oder die Mischung von Lösungsmitteln das erste Lösungsmittel oder die Mischung von Lösungsmitteln umfasst.

12. Nutzung zur Herstellung von Prothioconazol einer Mischung, die zwischen 1% w/w und 99% w/w eines Hydrazinderivats mit der Formel (I) umfasst, in einem ersten Lösungsmittel oder einer Mischung von Lösungsmitteln, wobei das erste Lösungsmittel oder die Mischung von Lösungsmitteln aus einem oder mehreren wasserunlöslichen Lösungsmitteln besteht

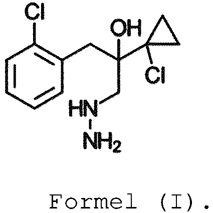

13. Ein Verfahren zur Herstellung eines Thionoderivats mit der Formel (III), das Folgendes umfasst:
(i) das Reagieren eines Oxirans mit der Formel (II) mit Hydrazin in der Anwesenheit von Wasser und einem ersten Lösungsmittel oder einer Mischung von Lösungsmitteln, wobei das erste Lösungsmittel oder die Mischung von Lösungsmitteln aus einem oder mehreren wasserunlöslichen Lösungsmitteln besteht, um eine Mischung herzustellen, die Folgendes umfasst: a) die neutrale Form eines Hydrazinderivats mit der Formel (I) und b) das erste Lösungsmittel oder die Mischung von Lösungsmitteln und
(ii) das Reagieren der Mischung, welche Folgendes umfasst: a) die neutrale Form eines Hydrazinderivats mit der Formel (I) und b) das erste Lösungsmittel oder die Mischung von Lösungsmitteln, resultierend aus Schritt (i), mit Formaldehyd und einem Thiocyanat mit der Formel X-SCN, worin X ein alkalisches Kation oder Ammonium ist; in Anwesenheit einer Säure und in Anwesenheit eines zweiten Lösungsmittels oder einer Mischung von Lösungsmitteln und Wasser, um das Thionoderivat mit der Formel (III) herzustellen, wobei das zweite Lösungsmittel oder die Mischung von Lösungsmitteln aus einem oder mehreren wasserunlöslichen Lösungsmitteln bestehen;
und wobei die Lösung Folgendes umfasst: a) die neutrale Form des Hydrazinderivats mit der Formel (I) und b) das erste Lösungsmittel oder die Mischung von Lösungsmitteln, resultierend aus Schritt (i), wird dem Schritt (ii) ohne Isolation als Mischung zugeführt, die zwischen 1% w/w und 99% w/w des Hydrazinderivats mit der Formel (I), bezogen auf das Gesamtgewicht der Lösung, umfasst;
wobei die Verbindungen mit den Formeln (I), (II) und (III) Folgende sind:

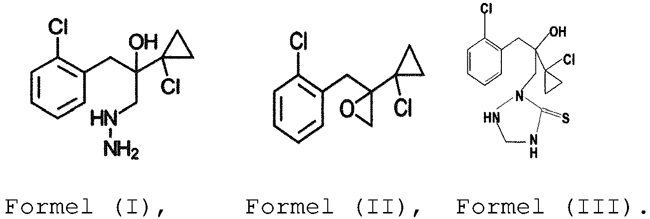

14. Ein Verfahren zur Herstellung eines Thionoderivats mit der Formel (III), das Folgendes umfasst:
(i) das Reagieren eines Oxirans mit der Formel (II) mit Hydrazin in Anwesenheit von Wasser und einem ersten Lösungsmittel oder einer Mischung von Lösungsmitteln, wobei das erste Lösungsmittel oder die Mischung von Lösungsmitteln aus einem oder mehreren wasserunlöslichen Lösungsmitteln besteht, um eine Mischung herzustellen, die die neutrale Form eines Hydrazinderivats mit der Formel (I) und das erste Lösungsmittel oder die Mischung von Lösungsmitteln umfasst;
(ia) das Trennen der zwei Phasen der Zweiphasenreaktionsmischung, um einen ersten Strom und einen zweiten Strom zu erzeugen, wobei der erste Strom wässeriges nicht umgesetztes Hydrazin umfasst und der zweite Strom das erste Lösungsmittel oder die Mischung von Lösungsmitteln und das Hydrazinderivat mit der Formel (I) umfasst; und
(ii) das Reagieren des zweiten Stroms mit Formaldehyd und einem Thiocyanat mit der Formel X-SCN, worin X ein alkalisches Kation oder Ammonium ist, in Anwesenheit einer Säure und in Anwesenheit eines zweiten Lösungsmittels oder einer Mischung von Lösungsmitteln und Wasser, um ein Thionoderivat mit der Formel (III) herzustellen, wobei das zweite Lösungsmittel oder die Mischung von Lösungsmitteln aus einem oder mehreren wasserunlöslichen Lösungsmitteln bestehen;
wobei der zweite Strom für Schritt (ii) ohne Isolation verwendet wird und eine Mischung von zwischen 1% w/w und 99% w/w des Hydrazinderivats mit der Formel (I) im ersten Lösungsmittel oder der Mischung von Lösungsmitteln, bezogen auf das Gesamtgewicht des zweiten Stroms, umfasst;
wobei die Verbindungen mit den Formeln (I), (II) und (III) die Folgenden sind:

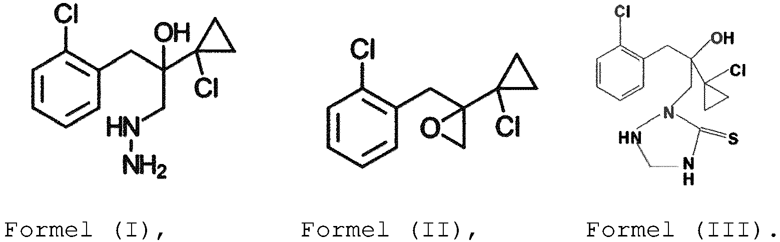

15. Ein Verfahren zur Herstellung von Prothioconazol, das Folgendes umfasst:
(i) das Reagieren eines Oxirans mit der Formel (II) mit Hydrazin in Anwesenheit von Wasser und einem ersten Lösungsmittel oder einer Mischung von Lösungsmitteln, wobei das erste Lösungsmittel oder die Mischung von Lösungsmitteln aus einem oder mehreren wasserunlöslichen Lösungsmitteln bestehen, um eine Mischung bereitzustellen, die die neutrale Form eines Hydrazinderivats mit der Formel (I) und das erste Lösungsmittel oder die Mischung von Lösungsmitteln umfasst;
(ii) das Reagieren des ersten Lösungsmittels oder der Mischung von Lösungsmitteln und des Hydrazinderivats mit der Formel (I), die aus Schritt (i) resultierten, mit Formaldehyd und einem Thiocyanat mit der Formel X-SCN, worin X ein alkalisches Kation oder Ammonium ist, in Anwesenheit einer Säure und in Anwesenheit eines zweiten Lösungsmittels oder einer Mischung von Lösungsmitteln und Wasser, um ein Thionoderivat mit der Formel (III) herzustellen, wobei das zweite Lösungsmittel oder die Mischung von Lösungsmitteln ein oder mehrere wasserunlösliche Lösungsmittel umfasst;
wobei die Mischung, die die neutrale Form des Hydrazinderivats mit der Formel (I) und das erste Lösungsmittel oder die Mischung von Lösungsmitteln, die aus Schritt (i) resultieren, umfasst, dem Schritt (ii) ohne Reinigung als Mischung zugeführt wird, die zwischen 1%w/w und 99%w/w des Hydrazinderivats mit der Formel (I) umfasst, bezogen auf das Gesamtgewicht der Mischung; und
(iii) das Umwandeln des Thionoderivats mit der Formel (III) in Prothioconazol; wobei die Verbindungen mit der Formel (I), (II) und (III) die folgenden sind:

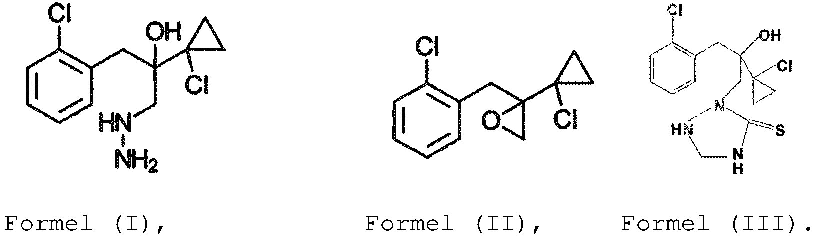

## Revendications

1. Procédé de préparation d'un mélange comprenant a) la forme neutre d'un dérivé d'hydrazine de formule (I) et b) un premier solvant ou mélange de solvants, le procédé comprenant (i) une réaction en deux phases entre un oxirane de formule (II) et l'hydrazine en présence d'eau et du premier solvant ou mélange de solvants, dans lequel ledit premier solvant ou mélange de solvants est constitué d'un ou plusieurs solvants insolubles dans l'eau ; et
dans lequel les composés de formule (I) et de formule (II) sont les suivants :

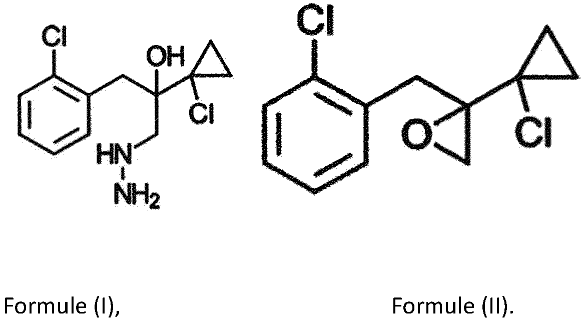

2. Procédé selon la revendication 1, dans lequel le ou les solvants insolubles dans l'eau comprennent un hydrocarbure aromatique en C₇-C₁₂.

3. Procédé selon la revendication 2, dans lequel l'hydrocarbure aromatique en C₇-C₁₂ est choisi dans le groupe constitué par le toluène, le xylène et leurs mélanges.

4. Procédé selon l'une quelconque des revendications précédentes, dans lequel la réaction a lieu en présence d'un catalyseur de transfert de phase.

5. Procédé selon la revendication 4, dans lequel le catalyseur de transfert de phase est choisi dans le groupe constitué par les sels de tétraalkylammonium, les sels de trialkyl aralkylammonium et les sels de tétraalkylphosphonium.

6. Procédé selon l'une quelconque des revendications 4 ou 5, dans lequel le catalyseur de transfert de phase est solide.

7. Procédé selon l'une quelconque des revendications précédentes, comprenant une étape supplémentaire (ia) dans laquelle les deux phases du mélange réactionnel en deux phases sont séparées pour créer un premier courant et un second courant, dans lequel le premier courant comprend de l'hydrazine aqueuse n'ayant pas réagi, et le second courant comprend ledit premier solvant ou mélange de solvants et le dérivé d'hydrazine de formule (I).

8. Procédé selon la revendication 1, dans lequel l'étape (ia) commence avant la fin de l'étape (i).

9. Procédé selon l'une quelconque des revendications 7 ou 8, dans lequel le premier courant comprend également un catalyseur de transfert de phase.

10. Procédé selon l'une quelconque des revendications 7 à 9, dans lequel le deuxième courant comprenant ledit premier solvant ou mélange de solvants et le dérivé d'hydrazine de formule (I) est introduit dans une réaction dans laquelle le dérivé d'hydrazine de formule (I) est transformé en un dérivé thiono de formule (III)

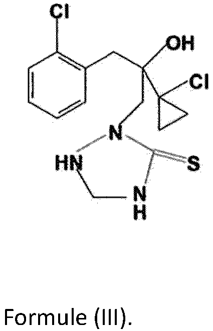

11. Procédé selon la revendication 10, dans lequel le dérivé d'hydrazine de formule (I) est traité en présence d'un second solvant ou mélange de solvants avec a) un thiocyanate de formule X-SCN, dans laquelle X est un cation alcalin ou l'ammonium, et b) du formaldéhyde, c) en présence d'un acide, dans lequel ledit second solvant ou mélange de solvants comprend le premier solvant ou mélange de solvants.

12. Utilisation pour préparer du prothioconazole d'un mélange comprenant entre 1 % en poids et 99 % en poids d'un dérivé d'hydrazine de formule (I) dans un premier solvant ou mélange de solvants, dans lequel ledit premier solvant ou mélange de solvants est constitué d'un ou plusieurs solvants insolubles dans l'eau.

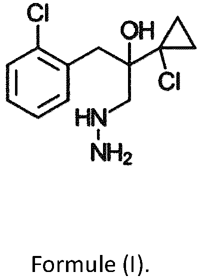

13. Procédé de préparation d'un dérivé thiono de formule (III) comprenant
(i) la réaction d'un oxirane de formule (II) avec de l'hydrazine, en présence d'eau et d'un premier solvant ou mélange de solvants, dans lequel ledit premier solvant ou mélange de solvants est constitué d'un ou plusieurs solvants insolubles dans l'eau, pour obtenir un mélange comprenant a) la forme neutre d'un dérivé d'hydrazine de formule (I) et b) le premier solvant ou mélange de solvants ; et
(ii) la réaction du mélange comprenant a) la forme neutre d'un dérivé d'hydrazine de formule (I) et b) le premier solvant ou mélange de solvants résultant de l'étape (i) avec du formaldéhyde et un thiocyanate de formule X-SCN, dans laquelle X est un cation alcalin ou l'ammonium, en présence d'un acide, et en présence d'un deuxième solvant ou mélange de solvants et d'eau, pour obtenir le dérivé thiono de formule (III), dans laquelle ledit deuxième solvant ou mélange de solvants est constitué d'un ou plusieurs solvants insolubles dans l'eau ;
dans lequel la solution comprenant a) la forme neutre du dérivé d'hydrazine de formule (I) et b) le premier solvant ou mélange de solvants résultant de l'étape (i) est introduite dans l'étape (ii) sans isolation sous forme d'un mélange comprenant entre 1 % en poids et 99 % en poids du dérivé d'hydrazine de formule (I), par rapport au poids total de la solution ;
dans lequel les composés de formule (I), formule (II) et formule (III) sont les suivants :

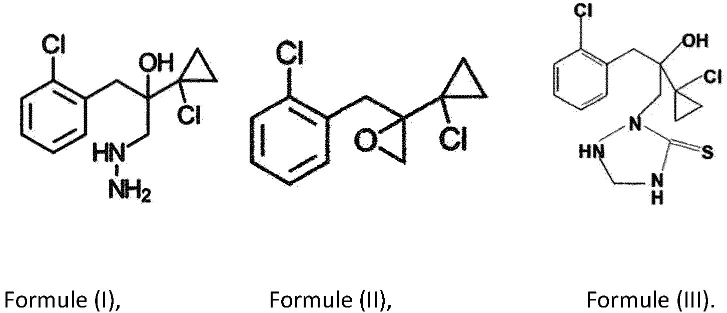

14. Procédé pour la préparation d'un dérivé thiono de formule (III), comprenant
(i) la réaction d'un oxirane de formule (II) avec de l'hydrazine, en présence d'eau et d'un premier solvant ou mélange de solvants, où ledit premier solvant ou mélange de solvants est constitué d'un ou plusieurs solvants insolubles dans l'eau, pour obtenir un mélange comprenant la forme neutre d'un dérivé d'hydrazine de formule (I) et le premier solvant ou mélange de solvants ;
(ia) la séparation des deux phases du mélange réactionnel à deux phases pour créer un premier courant et un second courant, dans lequel le premier courant comprend de l'hydrazine aqueuse n'ayant pas réagi, et le second courant comprend le premier solvant ou mélange de solvants et le dérivé d'hydrazine de formule (1) ; et
(ii) faire réagir le deuxième courant avec du formaldéhyde et un thiocyanate de formule X-SCN, dans laquelle X est un cation alcalin ou l'ammonium, en présence d'un acide et en présence d'un deuxième solvant ou mélange de solvants et d'eau pour obtenir un dérivé thiono de formule (III), dans laquelle ledit deuxième solvant ou mélange de solvants est constitué d'un ou plusieurs solvants insolubles dans l'eau ;
dans lequel le second courant est utilisé pour l'étape (ii) sans isolation et comprend un mélange compris entre 1 % en poids et 99 % en poids du dérivé d'hydrazine de formule (I) dans le premier solvant ou mélange de solvants, par rapport au poids total du second courant ;
dans laquelle les composés de formule (I), formule (II) et formule (III) sont les suivants :

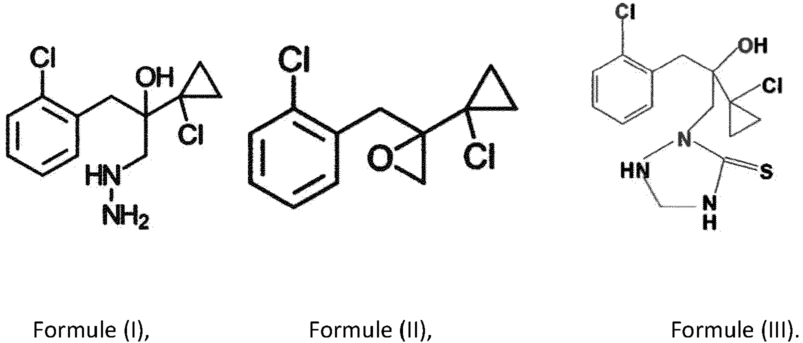

15. Procédé pour la préparation de prothioconazole comprenant
(i) la réaction d'un oxirane de formule (II) avec de l'hydrazine, en présence d'eau et d'un premier solvant ou mélange de solvants, ledit premier solvant ou mélange de solvants étant constitué d'un ou plusieurs solvants insolubles dans l'eau, pour obtenir un mélange comprenant la forme neutre d'un dérivé d'hydrazine de formule (I) et le premier solvant ou mélange de solvants ;
(ii) la réaction du premier solvant ou mélange de solvants et du dérivé d'hydrazine de formule (I) résultant de l'étape (i) avec du formaldéhyde et un thiocyanate de formule X-SCN, dans laquelle X est un cation alcalin ou l'ammonium, en présence d'un acide, et en présence d'un deuxième solvant ou mélange de solvants et d'eau pour obtenir un dérivé thiono de formule (III), dans laquelle ledit deuxième solvant ou mélange de solvants comprend un ou plusieurs solvants insolubles dans l'eau ;
dans lequel le mélange comprenant la forme neutre du dérivé d'hydrazine de formule (I) et le premier solvant ou mélange de solvants résultant de l'étape (i) est introduit dans l'étape (ii) sans purification sous forme d'un mélange comprenant entre 1 % en poids et 99 % en poids du dérivé d'hydrazine de formule (I), par rapport au poids total du mélange ; et
(iii) transformer le dérivé thiono de formule (III) en prothioconazole ;
où les composés de formule (I), formule (II) et formule (III) sont les suivants:
